# EUROPEAN PATENT APPLICATION

(11) **EP 3 143 871 A1**
(43) Date of publication of application: **22.03.2017**
(21) Application number: 15792594.2
(22) Date of filing: 15.05.2015
(51) Int. Cl.: A01G 17/00, A01G 31/00, A23D 9/02, B09B 3/00

(54) **PLANT PROCESSING SYSTEM**

(30) Priority: 15.05.2014 JP 2014101841
(71) Applicant: IHI Enviro Corporation, Tokyo 135-0042 (JP)
(72) Inventor: YAMASHITA, Masaharu, Tokyo 135-0042 (JP)
(74) Representative: Lamb, Martin John Carstairs
(86) International application number: PCT/JP2015/064029
(87) International publication number: WO 2015/174520

(57) **Abstract**

A plant processing system of the present invention includes: a plant cultivation facility (1) which cultivates plants including a sugar solution; a crushing facility (2) which crushes plants felled in the plant cultivation facility (1); a juicing facility (3) which harvests sap from plant chips obtained by the crushing facility (2); a methane fermentation facility (5) which performs a methane fermentation process on the sap; and a power generation facility (6) which generates electric power using a biogas obtained by the methane fermentation facility (5) as a fuel.

## Description

### [Technical Field]

The present invention relates to a plant processing system.

Priority is claimed on Japanese Patent application No. 2014-101841, filed on May 15, 2014, the content of which is incorporated herein by reference.

### [Background Art]

As known in the related art, palm oil is plant oil (a resultant object) which is produced by processing the fruit of the oil palm. The main production area of such palm oil is Southeast Asia, where it is produced on large-scale farms known as plantations. For example, Patent Document 1 and Patent Document 2 disclose that bioethanol (a liquid fuel) is produced by performing alcohol fermentation (ethanol fermentation) on sap (a saccharified liquid) serving as a raw material acquired from the trunk of an oil palm using the trunk as a biomass (a resource derived from organisms).

### [Document of Related Art]

### [Patent Document]

[Patent Document 1]
   Japanese Patent No. 4665257
[Patent Document 2]
   Japanese Patent No. 4418871

### [Summary of Invention]

### [Technical Problem]

However, since the techniques of Patent Document 1 and Patent Document 2 are techniques that focus on alcohol fermentation (ethanol fermentation), a relatively large amount of energy is required for producing bioethanol (a liquid fuel). There is a need to perform a process of concentrating sap (a sugar solution) as a step preceding alcohol fermentation, for example, to improve fermentation efficiency of alcohol fermentation or to prevent spoilage of the sap (the sugar solution) of an oil palm, and the concentrating process needs a great deal of energy. Also, a distillation process for separating bioethanol into simple substances is needed as a step subsequent to the alcohol fermentation, and the distillation process also needs a great deal of energy. When a distillation residue separated through a distillation process is used again as feed or the like, the distillation residue is a solid/liquid mixed solution containing a great amount of water. As such, a great deal of energy is needed to dry the distillation residue.

On the other hand, the techniques of Patent Document 1 and Patent Document 2 are techniques in which a palm trunk is attempted to be recycled as a biomass, but a biomass generated along with the production of palm oil (a resultant object) includes a palm trunk (a solid biomass) as well as a palm oil waste liquid (a liquid biomass) generated in an oil mill of palm oil. The palm oil waste liquid is drainage water (palm oil mill effect: POME) having a residue obtained by squeezing crude palm oil (CPO) from the fruit of an oil palm as a main component (sugar or the like) and is not effectively used as a biomass at present.

The present invention was made in view of the above-described circumstances and an object of the present invention is to improve an energy balance when a desired resultant object is produced by processing plants compared to the related art.

### [Solution to Problem]

A first aspect related to a plant processing system of the present invention includes: a plant cultivation facility which cultivates plants including a sugar solution; a crushing facility which crushes plants felled in the plant cultivation facility; a juicing facility which harvests sap from plant chips obtained by the crushing facility; a methane fermentation facility which performs a methane fermentation process on the sap; and a power generation facility which generates electric power using a biogas obtained by the methane fermentation facility as a fuel.

In a second aspect related to the plant processing system of the present invention, in the first aspect, the plant processing system further includes: a resultant object production facility which produces a predetermined resultant object from the plants; and a second methane fermentation facility which performs a methane fermentation process on a waste liquid generated in the resultant object production facility.

In a third aspect related to the plant processing system of the present invention, in the second aspect, the plant processing system further includes: a duckweed cultivation facility which performs a post-treatment on digestive juice generated in the second methane fermentation facility and cultivates a specific duckweed; and a fertilizer component recovery facility which recovers a fertilizer component from the duckweed cultivated in the duckweed cultivation facility.

In a fourth aspect related to the plant processing system of the present invention, in the second or third aspect, the methane fermentation facility and the second methane fermentation facility are integrally formed as a single facility.

In a fifth aspect related to the plant processing system of the present invention, in any one of the first to fourth aspects, the plant processing system further includes: a saccharification facility which performs a saccharification process on pulp of the plant chips obtained by the juicing facility, wherein the methane fermentation facility performs the methane fermentation process on saccharified liquid obtained by the saccharification facility as well as on the sap.

In a sixth aspect related to the plant processing system of the present invention, in any one of the first to fifth aspects, the plant processing system includes: a liquid fuelization facility which generates a liquid fuel from the biogas obtained by the methane fermentation facility instead of the power generation facility or in addition to the power generation facility.

In a seventh aspect related to the plant processing system of the present invention, in any one of the first to sixth aspects, one of the plants is an oil palm.

### [Effects of Invention]

According to the present invention, since electric power is acquired from a biogas generated by performing a methane fermentation process on sap obtained from plants, the electric power can be used as necessary electric power of the plant cultivation facility, the crushing facility, the juicing facility, fermentation facilities, and/or the power generation facility which generates the electric power. Therefore, according to the present invention, an energy balance when a desired resultant object is produced by processing plants can be improved compared to the related art.

### [Brief Description of Drawings]

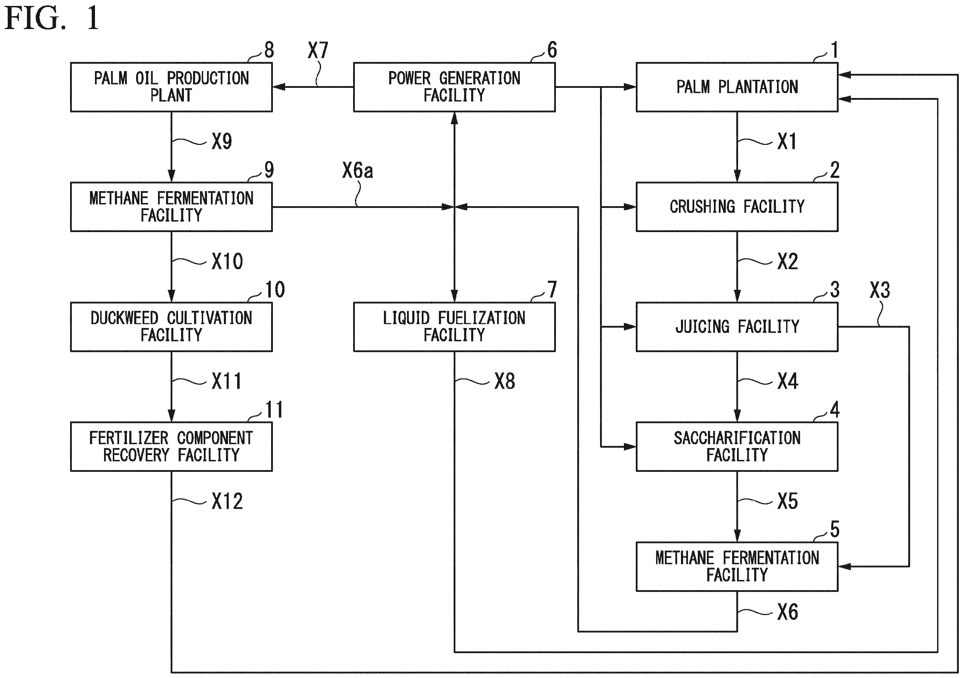

Fig. 1 is a view showing a constitution of a palm oil production system related to an embodiment of the present invention.

### [Description of Embodiments]

Hereinafter, an embodiment of the present invention will be described with reference to the accompanying drawings.

A palm oil production system related to the embodiment is a system (a plant processing system) which produces sap X3 and a saccharified liquid X5 by processing a trunk of an oil palm (a palm trunk X1) serving as a type of plant and produces palm oil serving as a type of plant oil by processing a fruit of the oil palm (a palm fruit). Note that, while the sap X3 and the saccharified liquid X5 in the palm oil production system are primary resultant objects obtained by processing oil palm, palm oil is a final object (a product).

As shown in Fig. 1, the palm oil production system related to the embodiment includes a palm plantation 1 (a plant cultivation facility), a crushing facility 2, a juicing facility 3, a saccharification facility 4, a methane fermentation facility 5, a power generation facility 6, a liquid fuelization facility 7, a palm oil production plant 8 (a resultant object production plant), a methane fermentation facility 9 (a second methane fermentation facility), a duckweed cultivation facility 10, and a fertilizer component recovery facility 11.

The palm plantation 1 among the facilities is a large scale plantation which cultivates oil palm. The oil palm is one of palm family plants which is mainly cultivated in Southeast Asia to produce palm oil, palm oil is harvested from a fruit (a palm fruit), and the palm fruit and a trunk (the palm trunk X1) contain a sugar solution. The palm plantation 1 ships a palm fruit to the palm oil production plant 8 and supplies a trunk (the palm trunk X1) obtained by felling an old oil palm tree to the crushing facility 2.

The crushing facility 2 is a facility which crushes the palm trunk X1 to a predetermined size of chips (palm chips X2). A size of the palm chips X2 (a chip size) are appropriately selected such that a juicing rate of the sap X3 in the juicing facility 3 of the subsequent stage is maximized. The crushing facility 2 supplies the palm chips X2 (plant chips) to the juicing facility 3.

The juicing facility 3 is a facility which juices the sap X3 from the palm chips X2. The sap X3 is a sugar solution having water and sugars (the simple sugars pentose and hexose) as main components. The juicing facility 3 supplies the sap X3 to the methane fermentation facility 5 of the subsequent stage and supplies pulp X4 (juice bagasse) of the sap X3 to the saccharification facility 4.

The saccharification facility 4 is a facility which performs low saccharification (monosaccharification) on the pulp X4. As known in the related art, a woody biomass such as the palm trunk X1 includes cellulose, hemicellulose, and lignin as its main components. The saccharification facility 4 hydrolyzes cellulose and hemicellulose among the main components of the palm trunk X1 in the presence of a saccharification enzyme. The saccharification facility 4 supplies the saccharified liquid X5 obtained by hydrolyzing the cellulose and hemicellulose to the methane fermentation facility 5.

Note that in "a saccharification process" in the saccharification facility 4, cellulose or hemicellulose need not necessarily be decomposed into a simple sugar or free sugar, but the pulp X4 (the juice bagasse) may be hydrolyzed to be liquefied or solubilized, and may decompose cellulose or hemicellulose may be decomposed into smaller units than simple sugars or free sugars in the process.

The methane fermentation facility 5 is a facility which performs methane fermentation on the sap X3 and the saccharified liquid X5. As known in the related art, methane fermentation is a reaction system in which organic matter is decomposed using an anaerobic organic matter decomposition process, that is, an action of methane bacteria serving as an anaerobic microorganism to generate a digestion gas having methane gas and carbon dioxide as its main components. The methane fermentation facility 5 provides a biogas X6 serving as a digestion gas to the power generation facility 6 and the liquid fuelization facility 7. Note that the methane fermentation facility 5 generates digestive juice as a drainage solution in addition to the biogas X6 (the digestion gas).

The power generation facility 6 is a facility which generates electric power using the biogas X6 supplied from the methane fermentation facility 5 and a biogas X6a supplied from the methane fermentation facility 9 (the second methane fermentation facility) as a fuel. In other words, the power generation facility 6 drives an electric power generator by operating a gas engine or a gas turbine using the biogases X6 and X6a as a fuel and supplies electric power X7 output from the electric power generator to the palm plantation 1, the crushing facility 2, the juicing facility 3, the saccharification facility 4, and/or the palm oil production plant 8.

The liquid fuelization facility 7 is a facility which converts the biogases X6 and X6a serving as a gas fuel into a liquid fuel X8. The liquid fuelization facility 7 generates the liquid fuel X8 by processing the biogases X6 and X6a on the basis of, for example, a Fischer-Tropsch (FT) method and supplies the liquid fuel X8 to the palm plantation 1 or the like.

The palm oil production plant 8 is a resultant object production facility which produces palm oil from a seed (the fruit) of an oil palm as a resultant object. In the palm oil production plant 8, a palm oil waste liquid X9 is generated in a process of producing the palm oil. The palm oil waste liquid X9 is drainage water (palm oil mill effect: POME) having crude palm oil (CPO) obtained by juicing the palm fruit as its main component and includes a sugar solution. The palm oil production plant 8 supplies the palm oil waste liquid X9 to the methane fermentation facility 9 (the second methane fermentation facility).

The methane fermentation facility 9 is a facility which generates the biogas X6a by performing a methane fermentation process on the palm oil waste liquid X9. In the methane fermentation facility 9, sugar in the palm oil waste liquid X9 is converted into the biogas X6a due to an action of methane bacteria, but components other than the sugar in the palm oil waste liquid X9 are components which constitute a digestive juice X10. The methane fermentation facility 9 provides the biogas X6a serving as a digestion gas to the power generation facility 6 and the liquid fuelization facility 7 and supplies the digestive juice X10 to the duckweed cultivation facility 10.

The duckweed cultivation facility 10 is a facility which purifies the digestive juice X10 and cultivates duckweed X11 on the basis of the digestive juice X10. The duckweed X11 absorbs and grows fertilizer components, that is, nitrogen (N), phosphorous (P), and potassium (K), included in the digestive juice X10 in a cultivation process. The duckweed cultivation facility 10 supplies the duckweed X11 which contains sufficient fertilizer components to the fertilizer component recovery facility 11. Note that a technique of absorbing a fertilizer component into duckweed (*wolffia globosa, lemna minor,* or *spirodela polyrhiza*) using the digestive juice of methane fermentation as a culture fluid is specifically disclosed in, for example, the specification of PCT International Publication No. WO2011/145316

The fertilizer component recovery facility 11 is a facility which produces fertilizer X12 from the duckweed X11. In other words, the fertilizer component recovery facility 11 acquires the fertilizer X12 by performing a predetermined process on the duckweed X11 and supplies the fertilizer X12 to the palm plantation 1.

Next, an overall operation of the palm oil production system configured in this way will described in greater detail.

The palm plantation 1 harvests and ships palm fruit to the palm oil production plant 8 if a plurality of oil palms are cultivated in a habitat (a field) of a predetermined area, and the palm fruit are grown up to a size which satisfy a predetermined shipment condition. There is a plurality of habitats having different growth years of oil palms in the palm plantation 1. Also, if an oil palm in the habitats becomes an old tree after a growth period of about 20 years has elapsed, a harvesting rate of the palm fruit is reduced. Thus, it is felled.

While this palm oil production system ships the palm fruit (a harvest) which is harvested in the palm plantation 1 to the palm oil production plant 8 to produce palm oil, the palm trunk X1 generated by felling an oil palm in the palm plantation 1 is supplied to the crushing facility 2 as a biomass. In other words, the crushing facility 2 crushes the log-shaped palm trunk X1 having, for example, a diameter of 30 to 60 cm and a height of about 10 m into the palm chips X2 (plant chips) having a maximum dimension of about 2.0 to 3.0 mm.

The juicing facility 3 separates the palm chips X2 into the sap X3 and solids (the pulp X4) using a predetermined squeezing device. Note that, before the palm chips X2 are juiced by the squeezing device, the palm chips X2 may be more finely ground using a cutter and/or the palm chips ground by the cutter may be crushed using a mill. The cutter is preferably a wet cutter, and the mill is preferably a wet mill.

A wet mill is, for example, a supermasscolloider (model number: MKZB-100J) manufactured by MASUKO SANGYO CO., LTD. Also, the wet mill may be a continuous vibrating mill (URAS TECHNO CO., LTD.). The continuous vibrating mill can continuously input and discharge granules without accumulating the granules.

The saccharification facility 4 generates the simple sugars pentose and hexose by hydrolyzing the pulp X4 on the basis of, for example, an enzyme saccharification method. As described above, the palm trunk X1 includes cellulose, hemicellulose, and lignin as main component. In an enzyme saccharifcation method, the cellulose and hemicellulose which are components of the palm trunk X1 are hydrolyzed in the presence of a saccharification enzyme. In other words, the saccharification facility 4 generates hexose by hydrolyzing the cellulose in the pulp X4 using activity of the saccharification enzyme and generates pentose by hydrolyzing the hemicellulose in the same pulp X4.

Simple sugars (pentose and hexose) generated by such hydrolysis are soluble in water, and therefore dissolve in water. In the above-described hydrolysis, solid/liquid mixed water including solids having lignin as a main component and the saccharified liquid X5 in which the simple sugars are dissolved in water, but the saccharification facility 4 separates only the saccharified liquid X5 from the solid/liquid mixed water and supplies the saccharified liquid X5 to the methane fermentation facility 5.

The saccharification process may be a microbial saccharification method using *Clostridium thermocellum.* In particular, the inventors of the present invention found that glucan and xylan can be decomposed at 62.5% and 39%, respectively, through a co-culture system of *Clostridizium thermocellum* and *Thermoanaerobacter brockii.* For this reason, the saccharification process can be performed through the co-culture system of *Clostridium thermocellum* and *Thermoanaerobacter brockii* with high efficiency.

The methane fermentation facility 5 generates the biogas X6 by performing methane fermentation on the sap X3 and the saccharified liquid X5. In other words, the methane fermentation facility 5 decomposes the sap X3 (the sugar solution) and the saccharified liquid X5 under an anaerobic environment due to an action of methane bacteria to generate the biogas X6 (the digestion gas) having methane gas and carbon dioxide as main components.

The power generation facility 6 drives an electric power generator by operating a gas engine or a gas turbine using the biogas X6 supplied from the methane fermentation facility 5 and the biogas X6a supplied from the methane fermentation facility 9 (the second methane fermentation facility) as a fuel. Also, the power generation facility 6 supplies electric power X7 generated by the electric power generator to the palm oil production plant 8, the palm plantation 1, the crushing facility 2, the juicing facility 3, the saccharification facility 4, etc.

The liquid fuelization facility 7 converts the biogases X6 and X6a serving as a gas fuel into the liquid fuel X8 such as synthetic naphtha by performing a synthesis gas generation process, an FT synthesis process, and an upgrade process. In other words, in the synthesis gas generation process, a mixed gas (a synthesis gas) of hydrogen gas (H₂) and carbon monoxide (CO) is generated from the biogases X6 and X6a. The biogases X6 and X6a are a mixed gas having methane (CH₄) and carbon dioxide (CO₂) as main components and are easily converted into synthesis gases using, for example, a steam reforming method, a partial oxidation method, etc.

Also, in the FT synthesis process, the synthesis gas is converted into mixed oil (FT synthetic oil) of hydrocarbons having various numbers of carbon atoms. The FT synthetic oil is mixed oil including, for example, methane, ethane, naphtha, kerosene, gas oil, etc. In the FT synthesis process, the FT synthetic oil is generated by heating the mixed gas (the mixed gas of hydrogen gas and carbon monoxide) in the presence of, for example, a metal catalyst, such as iron or cobalt.

In the upgrade process, the FT synthetic oil is subjected to a process distillation into fractions. In the upgrade process, for example, the FT synthetic oil is input into a distillation column and heated to be separated into fractions, and the fractions are cooled using water or the like, and thereby a liquid fuel X8 (a final product) such as synthetic naphtha, synthetic kerosene, and synthetic gas oil is acquired.

In the FT synthetic process and the upgrade process, drainage water is generated through a distillation process, but the drainage water includes an alcohol or an organic acid as a component. Therefore, the drainage water can be reprocessed (reused) using the methane fermentation facility 5, and thus there is no need to perform a separate drainage process.

The palm oil production plant 8 produces palm oil by performing a predetermined process on the palm fruit supplied from the palm plantation 1. In other words, the palm oil production plant 8 acquires CPO by purifying oil (squeezed oil) of the palm fruit through the squeezing device using heated water (hot water). Soluble components such as sugar in the squeezed oil and a suspension (solids) which are incorporated in the heated water when acquiring the crude palm oil are the palm oil waste liquid X9 and are generally referred to as a POME. The palm oil waste liquid X9 is a sugar solution which includes a large amount of sugar as one of the soluble components in the squeezed oil.

The methane fermentation facility 9 generates the biogas X6a (the digestion gas) by performing the methane fermentation process on the palm oil waste liquid X9. Also, the methane fermentation facility 9 supplies the biogas X6a to the power generation facility 6 and the liquid fuelization facility 7. In the methane fermentation facility 9, the digestive juice X10 is generated simultaneously with the biogas X6a (the digestion gas).

Here, when there are insufficient components which are needed for methane fermentation in the palm oil waste liquid X9, the methane fermentation is not efficiently performed. Thus, the insufficient components (for example, nickel, cobalt, molybdenum, etc.) can be appropriately added.

The duckweed cultivation facility 10 purifies the digestive juice X10 by performing an activated sludge process on the digestive juice X10 in a predetermined processing tank, and cultivates a specific duckweed X11 in the processing tank. The duckweed X11 is, for example, *wolffia globosa, lemna minor*, or *spirodela polyrhiza* and sufficiently absorbs the fertilizer components (nitrogen (N), phosphorous (P), and potassium (K)) included in the digestive juice X10 at a fully grown stage. Also, the fertilizer component recovery facility 11 acquires the fertilizer X12 by performing, for example, a drying process on the duckweed X11 and provides the fertilizer X12 to the palm plantation 1.

According to the embodiment, since the electric power X7 is acquired from the biogas X6 generated by performing the methane fermentation process on the sap X3 and the saccharified liquid X5 which are obtained from the palm trunk X1, the electric power X7 can be used as necessary electric power of the palm plantation 1, the crushing facility 2, the juicing facility 3, the saccharification facility and/or the palm oil production plant 8. Therefore, according to the embodiment, an energy balance in producing the palm oil (the final resultant object) can be improved compared to the related art by processing the palm fruit.

Also, according to the embodiment, since the liquid fuel X8 is acquired from the biogas X6 generated by performing the methane fermentation process on the sap X3 and the saccharified liquid X5 which are obtained from the palm trunk X1, the liquid fuel X8 can be used as necessary electric power of the palm plantation 1 or the like. Therefore, according to the embodiment, an energy balance in producing the palm oil (the final resultant object) can be improved compared to the related art by processing the palm fruit.

According to the embodiment, since the palm plantation 1 acquires the fertilizer X12 which is needed to cultivate the oil palm from the digestive juice X10 of the methane fermentation, an energy balance in producing the palm oil (the final resultant object) can also be accordingly improved compared to the related art by processing the palm fruit.

According to the embodiment, a palm oil waste liquid which has been discarded in the related art is used as a raw material of methane fermentation so that environmental impact of the palm oil production plant can be minimized.

According to the embodiment, the components in the digestive juice which has been discarded in the related art are recovered via duckweed and used again as a fertilizer so that environmental impact of the palm oil production plant can be minimized and resources can be effectively used compared to the related art.

Note that the present invention is not limited to the above-described embodiment, and, for example, the following modified examples are considered.
(1) The above-described embodiment relates to an oil palm from which palm oil is obtained, but the present invention is not limited thereto. A saccharified liquid and/or a biomass from which a saccharified liquid is obtained which are raw materials of methane fermentation include various wood biomasses or cellulose-based biomasses in addition to the oil palm. Therefore, the present invention can be applied to various woody biomasses or cellulose-based biomasses.
   For example, a cellulose-based biomass having sap containing sugar includes various plants such as palm leaves, bananas, sugar cane, corn, cassava, sago palm, yam, sorghum, potato, cellulose and sap (or juice), and crops including cellulose/starch/sap (or juice) in addition to the palm trunk X1, and the present invention can be applied to various cellulose-based biomasses.
(2) In the above-described embodiment, the palm oil waste liquid X9 is also used as a biomass in addition to the palm trunk X1, but the present invention is not limited thereto. Any one of the palm trunk X1 and the palm oil waste liquid X9 may be used as a biomass.
(3) In the embodiment, the electric power X7 and the liquid fuel X8 are obtained from the biogases X6 and X6a by providing the power generation facility 6 and the liquid fuelization facility 7, but the present invention is not limited thereto. Any one of the power generation facility 6 and the liquid fuelization facility 7 may be provided.
(4) In the embodiment, the saccharified liquid X5 is obtained from the pulp X4 by providing the saccharification facility 4, but the present invention is not limited thereto. The saccharification facility 4 may be omitted so that the biogas X6 is obtained using only sap X3 as a raw material.
(5) In the embodiment, fertilizer components are recovered from the digestive juice X10 of methane fermentation by providing the duckweed cultivation facility 10 and the fertilizer component recovery facility 11, but the present invention is not limited thereto. The duckweed cultivation facility 10 and the fertilizer component recovery facility 11 may be omitted as necessary.
(6) In the embodiment, the methane fermentation facility 5 and the methane fermentation facility 9 (the second methane fermentation facility) are individually provided, but the present invention is not limited thereto. The methane fermentation facility 5 and the methane fermentation facility 9 may be integrally formed as a single facility.

### [Industrial applicability]

According to the present invention, an energy balance in producing plants can be improved compared to the related art.

### [Description of Reference Signs]

- 1: Palm plantation (plant cultivation facility)
- 2: Crushing facility
- 3: Juicing facility
- 4: Saccharification facility
- 5: Methane fermentation facility
- 6: Power generation facility
- 7: Liquid fuelization facility
- 8: Palm oil production plant (resultant object production facility)
- 9: Methane fermentation facility (second methane fermentation facility)
- 10: Duckweed cultivation facility
- 11: Fertilizer (fertilizer component recovery facility)
- X1: Palm trunk
- X2: Palm chip (plant chip)
- X3: Sap
- X4: Pulp
- X5: Saccharified liquid
- X6, X6a: Biogas
- X7: Electric power
- X8: Liquid fuel
- X9: Palm oil waste liquid
- X10: Digestive juice
- X11: Duckweed
- X12: Fertilizer

## Claims

1. A plant processing system comprising:
a plant cultivation facility which cultivates plants including a sugar solution;
a crushing facility which crushes plants felled in the plant cultivation facility;
a juicing facility which harvests sap from plant chips obtained by the crushing facility;
a methane fermentation facility which performs a methane fermentation process on the sap; and
a power generation facility which generates electric power using a biogas obtained by the methane fermentation facility as a fuel.

2. The plant processing system according to claim 1, further comprising:
a resultant object production facility which produces a predetermined resultant object from the plants; and
a second methane fermentation facility which performs a methane fermentation process on a waste liquid generated in the resultant object production facility.

3. The plant processing system according to claim 2, further comprising:
a duckweed cultivation facility which performs a post-treatment on digestive juice generated in the second methane fermentation facility and cultivates a specific duckweed; and
a fertilizer component recovery facility which recovers a fertilizer component from the duckweed cultivated in the duckweed cultivation facility.

4. The plant processing system according to claim 2 or 3, wherein the methane fermentation facility and the second methane fermentation facility are integrally formed as a single facility.

5. The plant processing system according to any one of claims 1 to 4, further comprising:
a saccharification facility which performs a saccharification process on pulp of the plant chips obtained by the juicing facility,
wherein the methane fermentation facility performs the methane fermentation process on a saccharified liquid obtained by the saccharification facility as well as on the sap.

6. The plant processing system according to any one of claims 1 to 5, comprising:
a liquid fuelization facility which generates a liquid fuel from the biogas obtained by the methane fermentation facility instead of the power generation facility or in addition to the power generation facility.

7. The plant processing system according to any one of claims 1 to 6, wherein one of the plants is an oil palm.
